# FASCICULE DE BREVET EUROPEEN

(11) **EP 0 851 227 B1**
(45) Date de publication et mention de la délivrance du brevet: **21.06.2006**
(21) Numéro de dépôt: 97402782.3
(22) Date de dépôt: 24.12.1997
(51) Int. Cl.: G01N 33/50

(54) **Procédé d'évaluation des dommages induits dans la peau par UV-A**
Verfahren zur Bewertung der Schäden, die durch UV-A in der Haut hervorgerufen werden
Method for evaluating damage to the skin induced by UV-A

(30) Priorité: 24.12.1996 FR 9615987
(43) Date de publication de la demande: 01.07.1998
(73) Titulaire: L'ORÉAL, 75008 Paris (FR)
(72) Inventeur: Bernerd, Françoise, 75018 Paris (FR); Asselineau, Daniel, 92160 Antony (FR)
(74) Mandataire: Allab, Myriam

(56) Documents cités:
- EP-A- 0 358 506
- WO-A-91/16010
- FR-A- 2 689 904
- US-A- 3 967 124
- BERNERD F. ET AL: 'The sun protection factor (SPF) inadequately defines broad spectrum photoprotection : demonstration using skin reconstructed in vivo exposed to UVA, UVB or UV-solar simulated radiation' EUR. J. DERMATOL. vol. 13, no. 3, 2003, pages 242 - 249

## Description

L'invention a pour objet un procédé d'évaluation des dommages induits dans la peau et/ou le derme par les rayonnements ultraviolets de type A.

Le rayonnement solaire est composé, entre autres, de rayonnement ultraviolet de type A de longueurs d'onde comprises entre 320 nm et 400 nm (UV-A) et de rayonnements ultraviolets de type B de longueurs d'onde comprises entre 280 et 320 nm (UV-B).
Les UV-B sont fortement énergétiques et peu pénétrants, faiblement représentés dans la lumière solaire, dépendants des variations climatiques (temps nuageux, couvert, etc.) et leur présence varie en fonction des heures de la journée (notion de pic (zénith)).
Les UV-A sont moins énergétiques que les UV-B mais plus pénétrants, présents en grande quantité dans la lumière solaire (au minimum 100 fois plus d'UV-A que d'UV-B), peu dépendants des variations climatiques et présents quelle que soit l'heure de la journée.

Il est connu que le rayonnement solaire est responsable d'effets bénéfiques sur la peau, comme par exemple le brunissement, mais qu'il est aussi susceptible d'induire des dommages de celle-ci, notamment dans le cas d'une peau dite "sensible" ou d'une peau continuellement exposée.

Au titre des bienfaits le brunissement, communément appelé bronzage, est un élément essentiel du système de défense de la peau. En effet en réponse aux rayonnements ultraviolets, les mélanocytes de la couche supérieure de l'épiderme synthétisent de la mélanine qui une fois incorporée aux kératinocytes constitue un filtre situé à la surface de la peau, filtre qui absorbe le rayonnement ultraviolet Ceci a pour objectif de diminuer la quantité de rayonnement ultraviolet qui traverse les couches de la peau afin de l'empêcher d'atteindre les couches profondes et d'y causer des dommages néfastes pour la peau.

Au titre des effets nocifs, on sait qu'une exposition excessive de la peau aux rayonnements ultraviolets, aux rayonnements solaires en particulier, peut entraîner un changement dans l'élasticité de la peau, dans sa teneur en certains composés, et ainsi favoriser l'accélération du processus naturel de vieillissement de la peau. Ce processus de vieillissement accéléré ou prématuré dû aux UV est généralement appelé photo-vieillissement ou vieillissement actinique ou dermatohéliose.

Le photo-vieillissement résulte donc de l'action de facteurs extrinsèques sur la peau, parmi lesquels le rayonnement solaire, et en particulier les rayons U.V..
Le photo-vieillissement est donc la conséquence de la répétition d'événements définis, induits par ces facteurs extrinsèques, en particulier la rayonnement solaire, événements définis qui jusqu'à présent ont été supposés mais n'ont jamais été démontrés.
Par événements définis, on entend tout événement unique et ponctuel résultant d'une irradiation par les UVs.

Chez l'homme, le photo-vieiliissement fait que la peau présente un aspect clinique rugueux, sec, associé à une perte d'élasticité ainsi que des rides marquées.
Les signes histologiques du photo-vieillissement sont (pour revue *Gilchrest B.A. Skin and Aging Processes, 1989, CRC Press*) au niveau épidermique : la variation de l'épaisseur de l'épiderme (atrophie ou hyperplasie en fonction des zones observées), une atypie cellulaire (*Kligman L.M. and Kligman A.M., Photodermatol 1986, 3 : 215-227),* une perte de la polarité cellulaire, une irrégularité de la couche cornée, une diminution du nombre de cellules de Langerhans (*Lavker R.M. et col.*, *J. Invest. Dermatol. 1987, 88 : 44s-51s),* une pigmentation caractérisée par un aspect en mosaïque avec des zones d'hypo- ou d'hyper-pigmentation, une linéarisation de la jonction dermo-épidermique (*Lavker R.M., J. invest. Dermatol 1979, 73 59*-).
Les altérations épidermiques sont en fait relativement mineures en comparaison des altérations dermiques qui sont les plus flagrantes et les plus importantes *(Kligman L.M. and Kligman A.M., Photodermatol* 1986, *3 : 215-227*).
C'est en effet au niveau de la matrice extracellulaire que les principaux effets sont observés.. Les fibroblastes sont hyperactifs (*Kligman L.M. and Kligman A.M., Photodermatol 1986, 3* : *215-227)*. Le collagène est diminué en quantité (*Oikarinen A. et col., Photodermatol. 1985, 2 : 15-26, Warren R. et col., J. Am. Acad. Dermatol.* 1991, *25 : 757-760*). La solubilité des fibres est diminuée *(Oikarinen A. and Kallioinen M., Photodermatol. 1989, 6 : 24-31*), et on observe une dégénération basophile.
Les altérations ultrastructurales du collagène se traduisent par une diminution du nombre de fibrilles, une réduction de la densité électronique, une réduction des striations transversales (*Mitchell R.E., J. Invest. Dermatol. 1967, 48 : 203-220*). Inversement, le tissu élastique subit une hyperplasie (*Kligman A.M. J.A.M.A. 1969, 210 : 2377-2380, Warren R. et col., J. Am. Acad. Dermatol. 1991, 25 : 751-*760). Le derme moyen est caractérisé par un regroupement de fibres, formant ainsi une zone superficielle appelée "grenz zone", fibres qui sont absentes du derme papillaire. Dans la partie la plus profonde du derme, le tissu élastique est anormal et désorganisé (*Chen V.L. et col., J. Invest. Dermatol.* 1986, *87 : 334-337*), mais surtout il est présent en quantité importante ce qui se traduit histologiquement par un amas de tissu élastique. Ce phénomène porte le nom d'élastose actinique (*Braverman I.M. and Fonferko E., J. Invest. Dermatol. 1982*, *78 : 434-443 ; Matsuoka L. Y and Uitto J., Aging and the skin, Balin A.K and Kligman A.M. Eds, Raven Press N.Y. 1989, 7*, *141-151*) L'élastine présente un aspect granulaire (matériel élastotique) et des inclusions en microscopie électronique (*Braverman I.M, and Fonferko E., J. Invest. Dermatol. 1982, 78 : 434-*443). Les glycosaminoglycans et les protéoglycans sont augmentés (*Smith J.G. et col., J. Invest. Dermatol. 1962, 39 : 347-350, Sams W.M. and Smith J.G., J. Invest. Dermatol. 1961, 37: 447-452).*
Il a souvent été constaté la présence d'un infiltrat dermique composé de mastocytes, de lymphocytes et d'histiocytes (*Lavker R.M. and Kligman A.M., J. Invest. Dermatol, 1988, 90 : 325-330).* Les vaisseaux sanguins sont également modifiés (*Braverman I.M. and Fonferko E., J. Invest Dermatol. 1982, 78 : 444-448).* En particulier leur nombre est réduit, les cellules endothéliales sont dilatées et la paroi des vaisseaux est épaissie et on observe une lamination de la membrane basale des cellules endothéliales.

Cependant, même si les signes cliniques et histologiques d'une peau photovieillie ont été bien étudiés, les processus qui aboutissent à ces signes restent aujourd'hui inconnus. En particulier le rôle respectif des UV-A et des UV-B dans ces processus n'est pas démontré.
Cela est en grande partie dû à la difficulté de mettre en oeuvre un modèle d'étude simple et fiable.

On connaît dans l'art antérieur des modèles animaux développés dans le but de reproduire les dommages causés par les U.V. et dans un but pharmacologique pour tester des molécules "antivieillissement" *(Sams W.M, et col., J. Invest. Dermatol., 1964*, *43 : 467-471 ; Kligman L.H., Yearly review, the hairless mouse and photoaging Photochem. Photobiol., 1991,* 54(6), *1109-1118*).

Mais, ces modèles nécessitent l'utilisation d'animaux de laboratoires ce qui n'est pas sans poser des problèmes d'éthique.
De plus, pour obtenir une image de photo-vieillissement proche de la réalité il faut avec ces modèles pratiquer les irradiations de manière chronique et pendant plusieurs semaines ce qui alourdit la mise en oeuvre de tels modèles.
Les animaux utilisés dans ces modèles sont généralement des souris, dont les cellules sont différentes sur de nombreux points des cellules humaines.
De plus, la peau de !a souris est beaucoup plus fine que la peau humaine et ce paramètre est à considérer quand il s'agit des effets des rayonnements UV, car ces effets sont dépendants de la pénétration des rayons.
Le derme de la souris est différent du derme humain (derme papillaire et réticulaire inexistant chez la souris) et peut présenter selon l'espèce utilisée dans le modèle de nombreux résidus de follicules pileux dégénérés.
Enfin, ces modèles ne donnent pas d'indications précises sur les événements précoces qui aboutissent à l'image histologique du photo-vieillissement.

On connaît également des modèles d'étude chez l'homme (*Scharffetter K. et col., Arch. Dermatol. Res 1991, 283 506-511 ; Korwin-Zmijowska C. et col , Nouv. Dermatol. 1993*, *12 : 487*) mais on comprend aisément que leur mise en oeuvre soit difficile. L'utilisation de volontaires humains présente des difficultés d'ordre éthique et induit inévitablement des contraintes pour les sujets.
De plus, la variabilité entre individus (phototype de la peau, âge des sujets, antécédents, traitements en cours, ....) est telle que la reproductibilité des résultats est illusoire.

On connaît enfin des modèles *in vitro.* Certains de ces modèles font appel à des cultures cellulaires dans lesquelles on étudie les effets des irradiations U.V. sur un ou plusieurs marqueurs des cellules en culture.

Les effets des UVA ont ainsi été étudiés sur des kératinocytes (cellules épidermiques), des fibroblastes (cellules du derme), des cellules de Langerhans (cellules de l'épiderme immunocompétentes), ou des mélanocytes (cellules pigmentaires) (*Gilchrest B.A., J. Gerontol. 1980, 35 : 537-541 ; Scharffetter K. et col., Arch. Dermatol. Res. 1991, 283 506-511 ; Petersen M.J. et col., J*. *lnvest. Dermatol. 1992, 99 : 440-444 ; Nascimento A et col., Nucleic Acids Res., 1993, 21(5), 1103-1109).*
On comprend aisément que de tels modèles ne peuvent refléter la réalité du simple fait qu'il s'agit de cultures de cellules en monocouche ce qui est très différent de la notion de tissu et de son aspect tridimensionnel. La plupart du temps les cultures sont réalisées à partir d'une seule population cellulaire, ce qui ne reflète pas non plus la réalité.
Ainsi, l'influence de l'épaisseur des couches traversées par le rayonnement et le rôle de la pénétration des UVs dans la peau, bien évidemment fonction de l'épaisseur des couches cellulaires traversées, ne peuvent être étudiés dans de tels modèles.

L'absence de contexte matriciel, notamment en ce qui concerne les fibroblastes dermiques pour lesquels il est reconnu essentiel pour leur activité métabolique, fausse les interprétations des résultats obtenus ; il est en effet diffide d'extrapoler les données sur plastique avec une situation *in vivo* réelle chez l'homme

Il est impossible avec ce type de modèles d'effectuer des applications de produit par voie topique.
Enfin, certains de ces modèles font appel à des lignées cellulaires c'est-à-dire à des cellules génétiquement modifiées qui ne peuvent refléter la réalité physiologique de cellules normales.

On connaît aussi des modèles utilisant des équivalents d'épiderme ou de peau.
En ce qui concerne les modèles utilisant des équivalents d'épiderme, leur principal inconvénient vient du fait qu'ils ne représentent pas la réalité du fait de l'absence de derme ou d'équivalent de derme (*Noel-Hudson M.S. et col., Nouv. Dermatol., 1993, 12 : 493*)*.* On peut citer également dans cette ordre d'idée les tests de bronzage mis au point sur des équivalents d'épiderme contenant des mélanocytes (FR 2 689 904).

D'autres études utilisent des équivalents de peau *(Mammone. et col., J. Invest. Dermatol. 1992, 98, (4), 655 ; Ridge J.M. et col., Clin. Res., 1992, 40(2) : 543A Reece B. et col., J. Soc. Cosmet. Chem., 1992, 43 : 307-372 ; Pelie E. et col., J. Invest. Dermatol., 1993, 100, (4), 595, Nelson D. et col., Photochem. Photobiol. 1993, 57 (5) : 830-837); Haake and Polakowska, Cell death and differentiation. 1995, vol.2. 183-193.*
Indépendamment des modèles utilisés qui parfois sont assez éioignés d'une peau normale, ces études sont la plupart du temps restées très sommaires et n'ont en tout cas jamais étudié l'influence spécifique des UV-A sur des marqueurs corrélés au photo-vieillissement.
Ainsi, à ce jour aucun modèle d'étude n'a permis d'étudier et comprendre les événements dont la répétition aboutit aux signes cliniques et histologiques du photo-vieillissement.

La peau humaine est constituée de ceux compartiments à savoir un compartiment: superficiel, l'épiderme et un compartiment profond, le derme

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. Il est également constitué de vaisseaux sanguins et de fibres nerveuses. Dans une peau normale, c'est à dire non pathologique ni cicatricielle, le fibroblaste est à l'état quiescent, c'est à dire non prolifératif peu actif d'un point de vue métabolique et non mobile.

L'épiderme est la première cible atteinte par les rayonnements solaires, particulièrement les rayonnements UV.
Les UV-B faiblement pénétrants atteignent principalement l'épiderme. Le rôle des UV-B a été clairement démontré dans l'induction de cancers de ia peau UV-induits. Ils ont en effet comme principal chromophore les acides nucléiques en particulier l'acide désoxyribonucléique au sein duquel ils induisent des lésions et/ou des mutations (Eller M.S., 1995, in Photodamage. 26-56, Blackwell ed.)

A l'inverse, le rôle des UV-A dans l'induction des événements définis dont la répétition conduit au phénotype du photo-vieillissement n'est pas connu, même si leur fort pouvoir pénétrant laisse supposer qu'ils atteignent le derme dans lequel ils induisent leurs effets néfastes.

La demanderesse qui depuis fort longtemps s'intéresse à la peau et en particulier aux interactions des rayonnements solaires avec celle-ci a, après de longs travaux, maintenant découvert certains effets spécifiques des rayonnements UV de type A dans la peau, particulièrement dans le derme.

La demanderesse a pu montrer qu'une irradiation aux UV-A induit dans le derme des dégâts spécifiques aux longueurs d'ondes des UV-A. Ainsi, elle a pu montrer que les UV-A induisent la production de collagénase de type I par les fibroblastes, que le nombre desdits fibroblastes décroît dramatiquement du fait de l'irradiation jusqu'à une disparition totale dans les 48 heures suivants l'irradiation, tout au moins sur une épaisseur de derme qui est fonction des paramètres de l'irradiation et que la partie de derme d'où les fibroblastes ont disparu est recolonisée par des fibroblastes sous-jacents non atteints par le rayonnement. Ces trois événements sont directement liés à l'irradiation par les UV-A.

On comprend aisément que la production de collagénase (enzyme dégradant le collagène) dans une structure dont l'un des éléments essentiels est le collagène ne peut conduire qu'à des effets dramatiques pour la peau.

On comprend aussi que la disparition induite par les UV-A des fibroblastes, composants majeurs du derme et éléments régénérateurs de celui-ci, ne peut également conduire qu'à des effets dramatiques pour la peau.
Enfin, la recolonisation n'a lieu que si au moins une partie des "fibroblastes du derme n'est pas atteinte par le rayonnement. Cette réparation spontanée du dommage induit par les rayonnements, si elle présente un caractère favorable dans le sens qu'elle corrige les effets néfastes des rayonnements UV, n'en demeure pas moins une des causes du phénotype du photo-vieillissement. En effet, les fibroblastes doivent avoir quitté leur état de quiescence pour être aptes à recoloniser le tissus dermique. Cela implique qu'ils se multiplient, que leur métabolisme a repris et donc qu'ils se remettent à synthétiser les composants de la matrice extracellulaire dont une suraccumulation peut nuire à la peau.

Une peau qui subit les agressions répétées des rayonnements solaires et particulièrement de leur composante UV-A, qui subit donc une dégradation de son derme, est une peau qui semble vieillir précocement. La répétition de ces phénomènes fait que la peau présente les caractéristiques d'une peau photovieillie.

La découverte de ces marqueurs spécifiques de l'action des UV-A sur la peau, particulièrement sur le derme a conduit la demanderesse à mettre au point un modèle d'étude qui permet l'évaluation de produits susceptibles de moduler et/ou de corriger les dommages induits dans la peau et particulièrement dans le derme par les rayonnements ultraviolets de type A.

L'invention a pour objet un procédé d'évaluation des dommages induits dans la peau par les rayonnements ultraviolets de type A, caractérisé en ce que l'on soumet un équivalent de peau obtenu *in vitro* comprenant un équivalent de derme dont l'épaisseur est comprise entre 0,2 mm et 1,5 mm à au moins un rayonnement ultraviolet de type A pendant un temps suffisant, que l'on mesure la variation d'un marqueur spécifique des dommages induits dans !e derme par les rayonnements ultraviolets de type A et que l'on évalue les résultats de cette mesure par rapport à un témoin, ledit marqueur étant choisi parmi la concentration de collagénase de type I produite par les fibroblastes et le taux de recolonisation du derme par les fibroblastes sous-jacents.

Selon l'invention, tout équivalent de peau qui présente un équivalent de derme vivant dont l'épaisseur est comprise entre 0,2 mm et 1,5 mm est utilisable. Par équivalent de derme vivant, on entend tout équivalent de derme contenant des fibroblastes vivants. On citera à cet égard par exemple les équivalents de peau décrits dans les brevets ou dans les demandes de brevets EP 0285471, EP 0285474, EP 0418035, WO-A-90/02796. WO-A-91/16010. EP 0197090, EP 020753, CA 2119064.

Ainsi, l'invention présente de nombreux avantages liés à l'aspect tridimensionnel d'un équivalent de peau, à la présence des deux tissus (équivalent d'épiderme et équivalent de derme) qui permet d'étudier les interactions entre les deux tissus et le rôle de chaque type cellulaire dans la réponse à l'irradiation, à la nature et à l'origine des fibroblastes utilisés, à la composition de la matrice de départ, à la capacité de faire varier l'épaisseur de l'équivalent de derme, à la densité cellulaire variable de l'équivalent de derme, à la modification possible du milieu de culture utilisé pour obtenir l'équivalent de peau, à la capacité de faire varier la nature, la qualité, la quantité des rayonnements utilisés.

L'équivalent de derme utilisé selon l'invention peut être constitué par exemple de lattices mixtes collagène/fibroblastes, de substituts sous-cutanés à base de collagène contenant des fibroblastes, ou de tout autre support compatible avec la viabilité cellulaire dans lequel pourraient être inclus des fibroblastes.

Préférentiellement, l'équivalent de derme est constitué de lattices mixtes collagène/fibroblastes.

Les fibroblastes utilisés peuvent être de toute espèce (homme, souris, etc.) et de toute origine tissulaire (peau, poumon, etc.).
Les fibroblastes utilisés peuvent provenir de tissus sains ou pathologiques.
De préférence, on utilise des fibroblastes de peau humaine normale.

Mais, il est envisageable que le support comprenne en outre d'autres types cellulaires et/ou d'autres types de protéines. On citera par exemple les glycosaminoglycans ou les fibres d'élastine.

La densité de cellules utilisée est en général celle décrite dans l'art antérieur à propos des équivalents de peau obtenus *in vitro.* Mais, il est possible de faire varier cette densité en toute proportion que l'on estime être utile à l'évaluation objet du procédé de l'invention.

L'épaisseur de l'équivalent de derme utilisé selon l'invention est comprise entre 0,20 mm et 1,5 mm.

Il peut s'avérer intéressant de faire varier cette épaisseur en toute proportion que l'on estime être utile à l'évaluation objet du procédé de l'invention. L'homme du métier sait adapter la quantité de mélange collagène/cellule qu'il utilise pour obtenir l'équivalent de derme de l'épaisseur qu'il désire.

Selon l'invention, on soumet ledit équivalent de peau traité à un rayonnement ultraviolet de type A pendant un temps suffisant. Par un rayonnement ultraviolet de type A, on entend tout rayonnement présentant au moins les caractéristiques du rayonnement ultraviolet de type A, c'est à dire ayant au moins une longueur d'onde comprise entre 320 nm et 400 nm.
Ce rayonnement peut être le rayonnement solaire ou tout équivalent reproduit de manière artificielle.
De préférence selon l'invention, on utilise un rayonnement reproduit de manière artificielle ayant au moins une longueur d'onde comprise entre 320 nm et 400 nm et avantageusement un rayonnement ayant des longueurs d'onde uniquement comprises entre 320 nm et 400 nm. Les appareils reproduisant ce type de rayonnement sont bien connus. On peut citer à titre d'exemple le simulateur solaire IDEM 3000 de marque ARQUANTIEL ou encore le simulateur solaire de marque ORIEL. Quelque soit l'appareil choisi, celui-ci doit être équipé de filtres SCHOTT WG 335 3 mm qui éliminent les longueurs d'onde inférieures à 320 nm. On peut également utiliser des appareils du type lampes à bronzer comme par exemple les UVASUN 3000 donnés pour un spectre de longueurs d'onde supérieures à 340 nm.

A ce titre, l'invention présente l'avantage de permettre l'évaluation de la nature exacte du rayonnement émis par un matériel reproduisant le rayonnement solaire comme par exemple les lampes à bronzer. On sait qu'il est important avec un tel matériel que certaines longueurs d'onde ne soient pas émises afin de préserver la peau des effets néfastes qu'elles engendrent. Le procédé de l'invention peut permettre de parfaitement caractériser le rayonnement émis par l'évaluation des dommages causés par un tel matériel.

De manière générale, le temps pendant lequel on expose l'équivalent de peau obtenu *in vitro* traité au rayonnement de type UV-A est conditionné à la fois par les paramètres du rayonnement que l'on désire utiliser mais également par l'effet que l'on désire obtenir. C'est au minimum le temps nécessaire pour voir apparaître une modification du niveau d'expression du marqueur spécifique des dommages induits dans le derme par les rayonnements ultraviolets de type A.

Ce temps d'exposition au rayonnement peut aller de 1 minute à quelques heures. Préférentiellement, selon l'invention, le temps d'exposition est compris entre 5 et 120 minutes.

Selon l'invention, on mesure la variation d'un marqueur spécifique des dommages induits dans la peau, plus particulièrement dans le derme, par les rayonnements ultraviolets de type A et que l'on évalue les résultats de cette mesure par rapport à un témoin.

Par marqueur spécifique des dommages induits dans la peau, plus particulièrement dans le derme, par les rayonnements ultraviolets de type A, on entend selon l'invention, tout élément dont la présence, l'absence, la modification de l'expression ou la modification de la distribution peuvent être mesurées, en réponse à l'exposition au rayonnement de type A.

Plus particulièrement selon l'invention, le marqueur spécifique des dommages induits par les rayonnements ultraviolets de type A esf localisé dans le derme.

Comme on l'a vu précédemment, la demanderesse a pu montrer qu'une irradiation aux UV-A induit dans le derme des dégâts spécifiques des longueurs d'ondes des UV-A. Ainsi, elle a pu montrer que les UV-A induisent la production de collagénase de type I par les fibroblastes, ce qui se traduit par l'augmentation de la concentration de celle-ci dans le milieu. Elle a également montré que le nombre desdits fibroblastes décroît dramatiquement du fait de l'irradiation dans les 48 heures suivants l'irradiation, éventuellement jusqu'à une disparition totale, tout au moins sur une épaisseur de derme qui est fonction des paramètres de l'irradiation. Enfin elle a montré que la partie de derme d'où les fibroblastes ont disparu est recolonisée par des fibroblastes sous-jacents non atteints par le rayonnement.

Selon l'invention le marqueur spécifique des dommages induits dans le derme par les rayonnements ultraviolets de type A est la quantité de collagénase de type I par les fibroblastes et/ou le taux de recolonisation du derme par les fibroblastes sous-jacents.

Par taux de recolonisation, on entend le nombre de fibroblastes ayant recolonisé la partie de derme d'où les fibroblastes ont disparu après l'irradiation, et ceux au bout d'un temps choisi. Ce temps peut être compris entre 10 et 20 jours, de préférence entre 12 et 15 jours.

Les dommages induits dans le derme par les rayonnements ultraviolets de type A sont ainsi représentés par la variation du marqueur spécifique des dommages induits que l'on aura choisi de doser.
Par variation, on entend toute modification de la quantité, de la concentration, de la répartition du marqueur que l'on dose.

Pour cela, le procédé comme défini dans les revendications comprend une étape de dosage du marqueur spécifique des dommages induits.

Bien évidemment, quel que soit le mode de mise en oeuvre du procédé selon l'invention, toute méthode de dosage connue par l'homme du métier peut être utilisée.
On peut, à titre d'exemple et sans limitation, citer les méthodes de dosage des protéines ou des acides nucléiques par colorimétrie, par électrophorèse, par transcription reverse et/ou amplification par la technique des polymérisations en chaîne, par spectrographie' de masse, par chromatographie (en phase gazeuse ou sur plaque), les méthodes immunologiques, ou encore la microscope optique ou électronique et toutes les techniques histologiques, histochimiques et/ou immunohistochimiques.

Par exemple dans le cas de mesure de la production de collagénase de type I par les fibroblastes, on peut utiliser des techniques, classiques de biochimie permettant la détection de la collagénase de type I ou les techniques de biologie moléculaire ou encore les techniques d'immunohistochimie utilisant des anticorps dirigés contre la collagénase de type I. A cet égard, on peut citer les kits ELISA vendus par la société AMERSHAM.

Dans le cas de mesure du nombre de fibroblastes, les techniques de microscopie optiques et/ou électroniques, comme celles décrites dans Ganter et Jolles, Histochimie normale et pathologique, Gauthier-Villars éditeurs, Paris, 1969, sont particulièrement adaptées. Ces techniques sont utilisées selon les protocoles les plus classiques que l'on retrouve décrites dans les ouvrages. On peut citer par exemple la coloration histologique à l'hémalun-phloxine ou les techniques d'immunomarquage à l'aide d'anticorps permettant de mettre en évidence les fibroblastes.

Le résultat du dosage, qui représente la variation du marqueur spécifique des dommages induits que l'on a choisi de doser, n'est en soi pas exploitable directement. Il ne devient intéressant que dans la mesure où il est comparé à un témoin. Ce témoin est constitué par le résultat du même dosage effectué dans les mêmes conditions, mais en l'absence de toute irradiation ou en présence d'une irradiation par un rayonnement ayant des caractéristiques différentes.

L'homme du métier détermine aisément, par habitude, la nature du témoin nécessaire dans la mise en oeuvre du procédé.

De manière préférentielle, le témoin correspond à un équivalent de peau n'ayant subi aucune irradiation.

Le procédé selon l'invention peut ainsi permettre l'évaluation des dommages induits dans la peau par les rayonnements ultraviolets de type A Ceci est d'un grand intérêt dans l'optique de la connaissance des conséquences d'une irradiation de la peau par ce type de rayonnement et des mécanismes par lesquels la peau réagit et module ou corrige les effets de tels rayonnements.

Le procédé selon l'invention fait appel à un équivalent de peau qui en soi est un objet parfaitement manipulable. Grâce à l'art antérieur, on sait que l'équivalent de peau est cultivé dans un milieu de culture à l'interface air/liquide Il est donc envisageable d'ajouter au milieu de culture toute substance dont on veut évaluer l'influence sur les effets du rayonnement ultraviolet de type A Cela mime donc une application systémique de la substance.
Mais, dans la mesure où l'équivalent de peau présente une surface épidermique émergée, il est envisageable aussi d'appliquer sur cette surface toute substance dont on veut évaluer l'influence sur les effets du rayonnement ultraviolet de type A. Cela mime donc une application topique de la substance.

On a vu par ailleurs que la demanderesse a pu montrer que le nombre des fibroblastes dans le derme décroît dramatiquement dans les 48 heures suivants l'irradiation, éventuellement jusqu'à une disparition totale, tout au moins sur une épaisseur de derme qui est fonction des paramètres de l'irradiation. On a vu également que la demanderesse a mis en évidence que la partie de derme d'où les fibroblastes ont disparu est recolonisée par des fibroblastes sous-jacents non atteints par le rayonnement. Même si cette recolonisation spontanée peut nuire à la peau, il n'en demeure pas moins que, dans certaines circonstances, il peut être préférable de favoriser, voire de stimuler, cette réparation par l'application sur la peau de produits et/ou compositions sélectionnés pour ce type de propriété. Il est donc nécessaire, dans cette optique, de bénéficier d'un modèle d'étude simple et rapide d'évaluation de tels produits et/ou compositions.

Ainsi, le procédé selon l'invention peut en outre permettre l'évaluation de substances éventuellement susceptibles de moduler les dommages induits par les rayonnements ultraviolets de type A dans la peau et/ou le derme. Ainsi, le procédé selon l'invention comprend une étape supplémentaire de traitement de l'équivalent de peau obtenu *in vitro* avec un tel produit à tester.

Cette étape de traitement par un produit peut se situer avant ou après l'étape d'irradiation de l'équivalent de peau.

Le témoin dans ce cas peut être constitué par un équivalent de peau préalablement traité avec une quantité différente de la substance à tester ou encore par un équivalent de peau non traité par la substance à tester.
Préférentiellement, le témoin est constitué par un équivalent de peau non traité par la substance à tester.

De manière générale, le temps pendant lequel on traite l'équivalent de peau obtenu *in vitro* avec un produit à tester est conditionné par le temps qu'il faut à l'équivalent pour répondre à ladite substance, c'est à dire au temps qu'il faut pour qu'il produise ses effets.

Ce temps de traitement peut aller de quelques secondes à plusieurs jours.
A titre indicatif, le temps de traitement est généralement compris entre 5 minutes et 15 jours.

Par traitement de l'équivalent de peau obtenu *in vitro,* on entend une mise en contact de l'équivalent de peau obtenu *in vitro* avec la substance éventuellement susceptible de moduler les dommages induits par les rayonnements ultraviolets de type A.

La modulation du dommage induit par les rayonnements ultraviolets de type A peut correspondre à une diminution, voire à une suppression de ce dommage. Elle peut aussi correspondre à une inhibition de celui-ci. En d'autres termes ces substances peuvent être utilisées à titre préventif ou curatif.

La substance à tester peut être sous toute forme envisageable comme par exemple un composé éventuellement actif pur ou une composition éventuellement active ou contenant un composé éventuellement actif.

A ce titre, on peut citer par exemple les filtres solaires et/ou les compositions contenant de tels filtres. Dans ce cas, on préfère traiter l'équivalent de peau par simple application sur la surface dudit équivalent de peau.

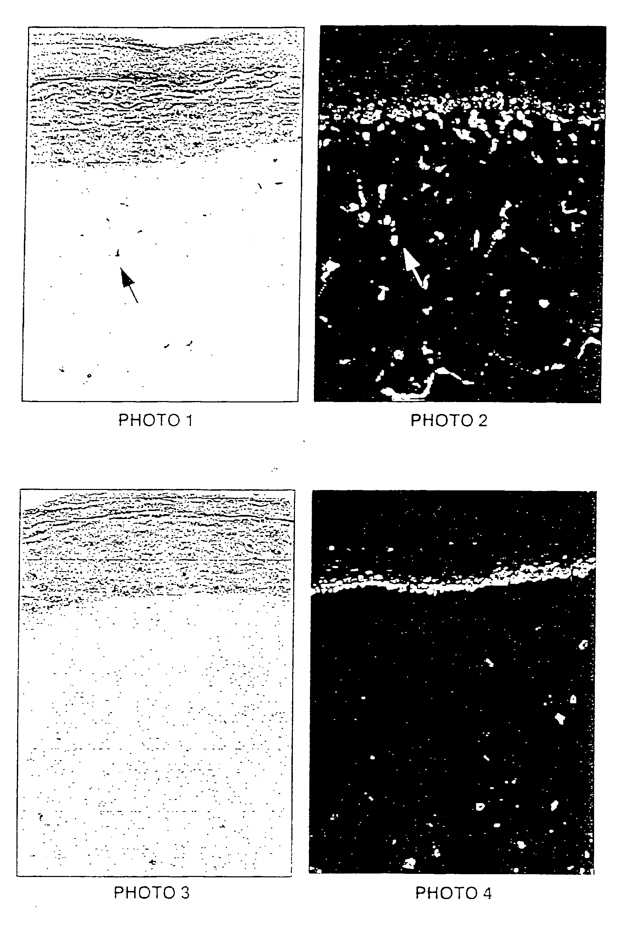

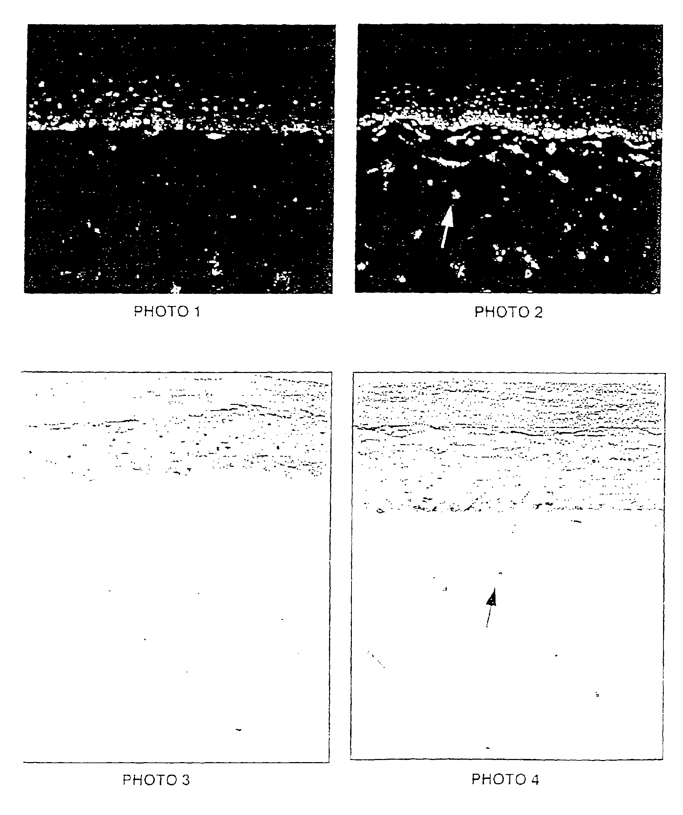

Les figures 1 et 2 permettent de mieux illustrer invention, sans toutefois en limiter sa portée
Dans la figure 1, les photos montrent des études des coupes des équivalents de peau en histologie classique (photos 1 et 3) ou en immunofluorescence (photos 2 et 4). Les photos 1 et 2 correspondent à un équivalent de peau non irradié, les photos 3 et 4 correspondent à l'équivalent de peau ayant subi une irradiation. , La flèche visualise les fibroblastes dans l'équivalent de derme.

Dans la figure 2, les photos montrent des études des coupes des équivalents de peau en histologie classique (photos 3 et 4) ou en immunofluorescence (photos 1 et 2).
Les photos 1 et 3 correspondent à un équivalent de peau préalablement traité avec un support seul, puis irradié. Les photos 2 et 4 correspondent à l'équivalent de peau préalablement traité avec le même support contenant un filtre à 5%, puis irradié.
La flèche visualise les fibroblastes dans l'équivalent de derme.

L'invention a également pour objet l'utilisation du procédé de l'invention pour évaluer la nature du rayonnement émis par un matériel reproduisant le rayonnement solaire comme les lampes à bronzer et/ou des dommages causés par un tel matériel.

On va maintenant donner, à titre indicatif et sans limitation aucune des exemples d'utilisation du procédé selon l'invention.

### Exemple 1 :

### Effet du rayonnement UV de type A sur la production de collagénase I :

### Protocole:

Equivalent de peau : les échantillons d'équivalents de peau sont préparés comme décrits dans Asselineau et col., Exp. Cell. Res. ,1985, 159, 536-539 ; Models in Dermatology, 1987, vol 3 pp 1-7. En résumé, les équivalents de derme sont préparés comme décrits avec du collagène de type I bovin, des fibroblastes issus de peau humaine normale adulte à raison de 10⁶ cellules pour un gel de 7 ml coulé dans une boîte de diamètre 60 mm. Ainsi préparé, après 3 jours de contraction le gel mesure 0,240 mm d'épaisseur. Les kératinocytes (50000) ensemencés sur cet équivalent de derme proviennent d'une plastie mammaire d'une femme de race blanche agée de 35 ans. La culture en immersion dure 7 jours et la culture à l'interface air-liquide également 7 jours. Le milieu de culture utilisé ne contient pas de rouge de phénol. A ce stade les peaux reconstruites ont été utilisées.

Irradiation: la source utilisée est un simulateur solaire de marque Arquantiel de modèle IDEM 3000 équipé d'une lampe Xénon de 1000 watts. Un filtre Schott UG11/1 mm et un filtre Schott WG 335 de 3mm éliminent les longueurs d'onde inférieures à 320 nm. Le spectre ainsi obtenu commence à 320 nm et couvre l'ensemble des rayonnements de type UV-A.
L'irradiance mesurée à l'aide d'un UV-mètre OSRAM équipé d'une sonde UV-A donne une valeur de 20 mW/cm².
Les doses délivrées sont de 20 J/cm² et 30 J/cm².
Les peaux reconstruites sont irradiées en l'absence de milieu de culture puis après le temps nécessaire à l'irradiation, les peaux reconstruites sont remises en culture émergée pendant 48 heures.
Le témoin est constitué par un équivalent d'épiderme identique ne subissant aucune irradiation (dose délivrée = 0 J/cm²).

Dosage de la collagénase interstitielle (type 1 ou MMP1) : 48 heures après l'irradiation, le milieu de culture est prélevé et la collagénase interstitielle est dosée à l'aide d'un kit "Biotrack MMP-1 human ELISA system" de marque Amersham (référence N°RPN 2610) selon le protocole préconisé par le fournisseur.
La lecture des résultats est réalisée à l'aide d'un lecteur de microplaques LabSystems équipé d'un filtre de 450 nm.
La quantité de collagénase I est calculée par rapport à une gamme étalon réalisée avec un standard fourni avec le kit, et est exprimée en ng/ml.

### Résultats et conclusion :

Le tableau ci-dessous montre qu'une irradiation UV-A de 20 J/cm² ou de 30J/cm² augmente la quantité de collagénase interstitielle dosée dans le milieu 48 heures après l'irradiation, en comparaison au témoin non irradié.

| Dose Collagénase Interstitielle (en J/cm²) (en ng/ml) | | |
|---|---|---|
| Témoin 0 | | 19,14 |
| Peau irradiée | 20 | 48,65 |
| Peau irradiée | 30 | 100, 27 |

### Exemple 2 :

### Effet du rayonnement UV de type A sur le nombre des fibroblastes du derme :

Un équivalent de peau identique à celui de l'exemple 1 est soumis à une irradiation de même source que dans l'exemple 1 à une dose de 25 J/cm².

48 heures après l'irradiation, l'équivalent de peau est coupée en deux.
Une moitié est préparée selon les techniques classiques d'histologie (Ganter et Jolles). Les coupes obtenues sont colorées à l'hémalun, phloxine, saphran (HPS) et observées à l'aide d'un microscope droit en lumière blanche.
L'autre moitié est incluse dans du tissu-Tek (Miles USA) et congelée en azote liquide. On prépare des coupes à congélation et on réalise des immunomarquages (selon protocole Bernerd et col., J. Invest. Dermatol., 1992, 98, 902-910) à l'aide d'un anticorps anti vimentine (Monosan, mouse monoclonal antibody anti human vimentin). La vimentine est un filament intermédiaire du cytosquelette des cellules mésenchymateuses dont font partie les fibroblastes (Traub P; 1985, in: intermediate filaments ; A review Springer-Verlag, Berlin). La révélation s'effectue à l'aide d'un second anticorps anti-lG de souris couplé à de la fluorescéine. L'observation se fait à l'aide d'un microscope à fluorescence.

Résultats et conclusion: L'histologie classique (figure 1, photos 1 et 3) ou l'immunofluorescence (figure 1, photos 2 et 4) montre que, 48 heures après l'irradiation UVA (25 J/cm²), les fibroblastes présents dans l'équivalent de derme non irradié (flèche, figure 1, photos 2 et 4) ont, du fait de l'irradiation, disparu dans la zone superficielle de celui-ci (figure 1, photos 1 et 3).

### Exemple 3 :

### Evaluation de l'efficacité de l'application de filtres solaires sur la peau :

Peaux reconstruites: même que pour exemple 1.

Un équivalent de peau identique à celui de l'exemple 1 préalablement traité avec est soumis à une irradiation de même source que dans l'exemple 1 à des doses de 0 J/cm² et de 25 J/cm².

On prépare un support (émulsion huile/eau) contenant un mélange alcool cétylstéarylique (80%) / alcool cétylstéarylique oxyéthyléné (33 oe) et monodistéarate de glycérol (7%), de l'huile minérale (15%), de la glycérine (20%), et de l'eau (qsp). On prépare à partir de ce support une composition contenant un filtre solaire, le Mexoryl SX (brevet FR 82-10425), à 5%. On applique le support ou la composition contenant le filtre dans le support à raison de 2 mg/cm² à la surface de l'équivalent de peau. Les échantillons sont ensuite irradiés comme dans l'exemple 1, puis le produit est rincé 3 fois avec du tampon phosphate stérile (Seromed L 1825) et les équivalents de peau sont remis en culture, à l'interface air-liquide pendant 48 heures.

Visualisation de la disparition des fibroblastes dans le derme équivalent: Telle que décrite dans l'exemple 2

Résultats et conclusion : 48 heures après l'irradiation (UVA 25 J/cm²), les fibroblastes présents dans l'équivalent de derme ont disparu dans la zone superficielle du derme dans le cas de l'équivalent de peau préalablement traité avec le support, ce qui correspond aux effets induits par les UVA illustrés dans l'exemple 2 (figure 2, photos 1 et 3; En comparaison dans l'équivalent de peau préalablement traité avec la composition contenant le filtre à 5% (figure 2, photos 2 et 4), les fibroblastes ne disparaissent pas (flèche) ce qui témoigne d'une protection apportée par le filtre par rapport aux dommages induits par les UVA.

## Revendications

1. Procédé d'évaluation des dommages induits dans la peau par les rayonnements ultraviolets de type A, **caractérisé en ce que** l'on soumet un équivalent de peau obtenu *in vitro* comprenant un équivalent de derme dont l'épaisseur est comprise entre 0,2 mm et 1,5 mm à au moins un rayonnement ultraviolet de type A pendant un temps suffisant, que l'on mesure la variation d'un marqueur spécifique des dommages induits dans la peau par les rayonnements ultraviolets de type A dans le derme,
ledit marqueur étant choisi parmi la concentration de collagénase de type I produite par les fibroblastes, et le taux de recolonisation du derme par les fibroblastes sous-jacents,
et que l'on évalue les résultats de cette mesure par rapport à un témoin.

2. Procédé selon l'une quelconque des revendications précédentes, **caractérisé en ce que** l'équivalent de peau est constitué d'un équivalent d'épiderme et d'un équivalent de derme vivant.

3. Procédé selon l'une quelconque des revendications précédentes, **caractérisé en ce que** le rayonnement ultraviolet de type A a au moins une longueur d'onde comprise entre 320 nm et 400 nm.

4. Procédé selon l'une quelconque des revendications précédentes, **caractérisé en ce que** le traitement par le rayonnement ultraviolet de type A dure un temps compris entre 1 minute à quelques heures.

5. Procédé selon la revendication précédente, **caractérisé en ce que** le traitement par le rayonnement ultraviolet de type A dure un temps compris entre 5 et 120 minutes.

6. Procédé selon l'une quelconque des revendications précédentes, **caractérisé par le fait que** le marqueur spécifique des dommages induits par les rayonnements ultraviolets de type A est une cellule ou une population cellulaire, un acide nucléiqué, une protéine ou un groupe de protéines liées ou non, un ion organite cellulaire, un lipide, ou un polyoside.

7. Procédé selon l'une quelconque des revendications précédentes, **caractérisé par le fait que** le témoin est constitué par le résultat du même dosage effectué dans les mêmes conditions, mais en l'absence de toute irradiation ou en présence d'une irradiation par un rayonnement ayant des caractéristiques différentes.

8. Procédé selon la revendication précédente, **caractérisé en ce que** le témoin est constitué par un équivalent de peau n'ayant subi aucune irradiation.

9. Procédé pour évaluer une substance susceptible de moduler les dommages induits dans la peau par les rayonnements ultraviolets de type A, **caractérisé par le fait que** dans le procédé des revendications 1 à 8 on traite un équivalent de peau avec ladite substance pendant un temps suffisant avant ou après l'irradiation de J'équivalent de peau par les rayonnements ultraviolets de type A.

10. Procédé selon la revendication 9, **caractérisé par le fait que** le témoin est constitué par un équivalent de peau préalablement traité avec une quantité différente de la substance à tester ou encore par un équivalent de peau non traité par la substance à tester.

11. Procédé selon la revendication précédente, **caractérisé en ce que** le témoin est constitué par un équivalent de peau non traité par la substance à tester.

12. Procédé selon l'une quelconque des revendications 9 à 11, **caractérisé en ce que** l'on traite l'équivalent de peau pendant un temps compris entre quelques secondes et plusieurs jours.

13. Procédé selon la revendication précédente, **caractérisé en ce que** l'on traite l'équivalent de peau pendant un temps compris entre 5 minutes et 15 jours.

14. Procédé pour caractériser le rayonnement de type UVA émis par un matériel reproduisant le rayonnement solaire et/ou des dommages causés par un tel rayonnement, **caractérisé en ce que** l'on soumet un équivalent de peau obtenu in vitro audit rayonnement et **en ce qu'**on évalue par le procédé selon l'une des revendications 1 à 8 si les dommages induits dans ledit équivalent de peau sont ceux induits par les rayonnements ultraviolets de type A.

## Claims

1. Process for evaluating the damage induced in skin by type A ultraviolet radiation, **characterized in that** a skin equivalent obtained *in vitro* comprising a dermis equivalent with a thickness between 0.2mm and 1.5 mm is subjected to at least a type A ultraviolet radiation for a sufficient time, **in that** the variation of a marker specific for the damage induced in the skin by the type A ultraviolet radiation in the dermis is measured,
the said marker being selected from the concentration of type I collagenase produced by the fibroblasts and the level of recolonization of the dermis by the subjacent fibroblasts,
and **in that** the results of this measurement are evaluated relative to a control.

2. Process according to any one of the preceding claims, **characterized in that** the skin equivalent consists of an epidermis equivalent and of a living dermis equivalent.

3. Process according to any one of the preceding claims, **characterized in that** the type A ultraviolet radiation has at least a wavelength of between 320 nm and 400 nm.

4. Process according to any one of the preceding claims, **characterized in that** the treatment by the type A ultraviolet radiation lasts for a time between 1 minute and a few hours.

5. Process according to the preceding claim, **characterized in that** the treatment by the type A ultraviolet radiation lasts for a time between 5 and 120 minutes.

6. Process according to any one of the preceding claims, **characterized in that** the marker specific for the damage induced by the type A ultraviolet radiation is a cell, a cell population, a nucleic acid, a protein, a group of proteins, bound or otherwise, an ion, a cellular organelle, a lipid or a polysaccharide.

7. Process according to any one of the preceding claims, **characterized in that** the control consists of the result of the same assay carried out under the same conditions, but in the absence of any irradiation or in the presence of irradiation with radiation having different characteristics.

8. Process according to the preceding claim, **characterized in that** the control consists of a skin equivalent which has not been subjected to any irradiation.

9. Process for evaluating a substance capable of modulating the damage induced in the skin by the type A ultraviolet radiation, **characterized in that** in the process of Claims 1 to 8, a skin equivalent is treated with the said substance for a sufficient time before or after the irradiation of the skin equivalent with the type A ultraviolet radiation.

10. Process according to Claim 9, **characterized in that** the control consists of a skin equivalent previously treated with a different quantity of the substance to be tested or of a skin equivalent not treated with the substance to be tested.

11. Process according to the preceding claim, **characterized in that** the control consists of a skin equivalent not treated with the substance to be tested.

12. Process according to any one of Claims 9 to 11, **characterized in that** the skin equivalent is treated for a time between a few seconds and several days.

13. Process according to the preceding claim, **characterized in that** the skin equivalent is treated for a time between 5 minutes and 15 days.

14. Process for characterizing the UVA type radiation emitted by equipment reproducing solar radiation and/or damage caused by such radiation, **characterized in that** a skin equivalent obtained *in vitro* is subjected to the said radiation and **in that** it is evaluated, by the process according to one of Claims 1 to 8, whether the damage induced in the said skin equivalent is that induced by the type A ultraviolet radiation.

## Patentansprüche

1. Verfahren zur Bewertung der Schäden, die in der Haut durch Ultraviolettstrahlung vom Typ A hervorgerufen werden, **dadurch gekennzeichnet,**
**dass** ein *in vitro* erhaltenes Hautäquivalent, das ein Dermisäquivalent umfasst, dessen Dicke im Bereich von 0,2 bis 1,5 mm liegt, mindestens einer Ultraviolettstrahlung vom Typ A über einen ausreichenden Zeitraum ausgesetzt wird,
**dass** die Änderung eines Markers gemessen wird, der spezifisch für die Schäden ist, die in der Haut durch Ultraviolettstrahlung vom Typ A in der Dermis hervorgerufen werden, wobei der Marker unter der Konzentration der Kollagenase vom Typ I, die von den Fibroblasten erzeugt wird, und dem Ausmaß der Neubesiedlung der Dermis mit darunter liegenden Fibroblasten ausgewählt wird, und
**dass** die Ergebnisse dieser Messung, bezogen auf einen Vergleich, bewertet werden.

2. Verfahren nach einem der vorhergehenden Ansprüche, **dadurch gekennzeichnet, dass** das Hautäquivalent aus einem lebenden Epidermisäquivalent und einem lebenden Dermisäquivalent besteht.

3. Verfahren nach einem der vorhergehenden Ansprüche, **dadurch gekennzeichnet, dass** die Ultraviolettstrahlung vom Typ A mindestens eine Wellenlänge aufweist, die im Bereich von 320 bis 400 nm liegt.

4. Verfahren nach einem der vorhergehenden Ansprüche, **dadurch gekennzeichnet, dass** die Behandlung mit der Ultraviolettstrahlung vom Typ A über einen Zeitraum durchgeführt wird, der im Bereich von einer Minute bis mehreren Stunden liegt.

5. Verfahren nach dem vorhergehenden Anspruch, **dadurch gekennzeichnet, dass** die Behandlung mit der Ultraviolettstrahlung vom Typ A über einen Zeitraum durchgeführt wird, der im Bereich von 5 bis 120 min liegt.

6. Verfahren nach einem der vorhergehenden Ansprüche, **dadurch gekennzeichnet, dass** der Marker, der für die Schäden spezifisch ist, die durch Ultraviolettstrahlung vom Typ A hervorgerufen werden, eine Zelle oder eine Zellpopulation, eine Nucleinsäure, ein Protein oder eine Gruppe von zusammenhängenden oder nicht zusammenhängenden Proteinen, ein zelluläres organisches Ion, ein Lipid oder ein Polysaccharid ist.

7. Verfahren nach einem der vorhergehenden Ansprüche, **dadurch gekennzeichnet, dass** der Vergleich aus dem Ergebnis der gleichen quantitativen Bestimmung besteht, die unter den gleichen Bedingungen durchgeführt wurde, jedoch in Abwesenheit jeglicher Bestrahlung oder in Gegenwart einer Bestrahlung mit einer Strahlung, die andere Eigenschaften hat.

8. Verfahren nach dem vorhergehenden Anspruch, **dadurch gekennzeichnet, dass** der Vergleich aus einem Hautäquivalent besteht, das keiner Bestrahlung unterzogen wurde.

9. Verfahren zur Bewertung einer Substanz, die eventuell die Schäden modulieren kann, die in der Haut durch Ultraviolettstrahlung vom Typ A hervorgerufen werden, **dadurch gekennzeichnet, dass** in dem Verfahren der Ansprüche 1 bis 8 ein Hautäquivalent vor oder nach der Bestrahlung des Hautäquivalents mit Ultraviolettstrählung vom Typ A über einen ausreichenden Zeitraum mit der Substanz behandelt wird.

10. Verfahren nach Anspruch 9, **dadurch gekennzeichnet, dass** der Vergleich aus einem Hautäquivalent, das vorab mit einer anderen Menge der zu prüfenden Substanz behandelt wurde, oder aus einem Hautäquivalent, das nicht mit der zu prüfenden Substanz behandelt wurde, besteht.

11. Verfahren nach dem vorhergehenden Anspruch, **dadurch gekennzeichnet, dass** der Vergleich aus einem Hautäquivalent besteht, das nicht mit der zu prüfenden Substanz behandelt wurde.

12. Verfahren nach einem der Ansprüche 9 bis 11, **dadurch gekennzeichnet, dass** das Hautäquivalent über einen Zeitraum behandelt wird, der im Bereich von einigen Sekunden bis mehreren Tagen liegt.

13. Verfahren nach dem vorhergehenden Anspruch, **dadurch gekennzeichnet, dass** das Hautäquivalent über einen Zeitraum behandelt wird, der im Bereich von 5 min bis 15 Tagen liegt.

14. Verfahren zum Charakterisieren der Strahlung vom Typ UV-A, die von einem Material emittiert wird, dass die Sonnenstrahlung reproduziert, und/oder der Schäden, die durch eine derartige Strahlung verursacht werden, **dadurch gekennzeichnet, dass** ein *in vitro* erhaltenes Hautäquivalent der Strahlung ausgesetzt wird und dass durch das Verfahren nach einem der Ansprüche 1 bis 8 bewertet wird, ob die in diesem Hautäquivalent hervorgerufenen Schäden die Schäden sind, die durch Ultraviolettstrahlung vom Typ A hervorgerufen werden.
